# FASCICULE DE BREVET EUROPEEN

(11) **EP 1 622 671 B1**
(45) Date de publication et mention de la délivrance du brevet: **23.07.2008**
(21) Numéro de dépôt: 04742717.4
(22) Date de dépôt: 13.05.2004
(51) Int. Cl.: A61M 25/06

(54) **ENSEMBLE COMPRENANT UNE AIGUILLE DE PONCTION ET UN TUBE PROTECTEUR D’AIGUILLE ET APPLICATION**
ANORDNUNG BESTEHEND AUS EINER PUNKTIONSNADEL UND EIN NADELSCHUTZROHR UND VERWENDUNG
ASSEMBLY COMPRISING AN ASPIRATING NEEDLE AND A NEEDLE PROTECTOR TUBE, AND APPLICATION THEREOF

(30) Priorité: 14.05.2003 FR 0305771
(43) Date de publication de la demande: 08.02.2006
(73) Titulaire: VYGON, F-95440 Ecouen (FR)
(72) Inventeur: CARREZ, Jean-Luc, F-95440 Ecouen (FR); DALLE, Valéry, F-60270 Gouvieux (FR); GUYOMARC'H, Pierrick, F-95120 Ermont (FR)
(74) Mandataire: Texier, Christian
(86) Numéro de dépôt international: PCT/FR2004/001163
(87) Numéro de publication internationale: WO 2004/101056

(56) Documents cités:
- EP-A- 0 545 671
- EP-A- 0 578 367
- EP-A- 0 875 261
- US-B1- 6 500 129

## Description

L'invention concerne un ensemble constitué d'une aiguille de ponction et d'un tube protecteur d'aiguille, et une application de cet ensemble à la mise en place d'un cathéter court.

Elle concerne plus particulièrement un ensemble comprenant une aiguille de ponction constituée d'un tube d'aiguille dont une extrémité est biseautée et d'une embase solidaire de l'autre extrémité du tube d'aiguille, et un tube protecteur pour cette aiguille, ce tube protecteur présentant une fente longitudinale qui permet de faire coulisser le tube d'aiguille dans le tube protecteur en actionnant une partie de l'aiguille extérieure au tube protecteur.

A titre d'exemple connu d'un tel ensemble, on citera la réalisation décrite dans la publication US 5 954 698. le document us 6 500 129 décrit aussi un tel ensemble.

L'invention a pour objet un ensemble aiguille/tube protecteur caractérisé en ce que le tube d'aiguille comporte une portion de tube rectiligne dont une extrémité est biseautée et dont l'autre extrémité se raccorde par une portion coudée à une portion oblique du tube qui aboutit dans l'embase d'aiguille, en ce que le tube protecteur est un tube souple de diamètre apte à loger intégralement ladite portion rectiligne du tube d'aiguille, tandis que ladite embase de l'aiguille reste intégralement située à l'extérieur du tube protecteur et est montée à coulisse sur le tube protecteur, entre une position avant où ladite portion rectiligne est en saillie en avant du tube protecteur et une position arrière où ladite portion rectiligne est contenue dans le tube protecteur, et en ce que ladite fente longitudinale du tube protecteur s'étend jusqu'à l'extrémité avant du tube protecteur et étant à ouverture élastique de façon à s'ouvrir au passage de la portion coudée du tube d'aiguille au travers de la fente et à se refermer après ce passage.

Dans des modes de réalisation préférés, l'ensemble de l'invention présente encore une ou plusieurs des caractéristiques supplémentaires suivantes :
- Le tube protecteur est déformable pour ne pas gêner la prise en main de l'ensemble lors de la ponction et il est muni à l'avant d'une pièce rapportée rigide pour son raccordement à l'embase du cathéter. « Déformable » signifie que le tube n'est pas rigide et peut être courbé à la demande, si nécessaire, pour ne pas gêner les manipulations notamment lors de la ponction et de l'avancement de la canule ; de préférence il est élastiquement déformable.
   On connaît déjà des protecteurs aptes à protéger tout le tube d'aiguille mais ces protecteurs sont des tubes rigides et encombrants qui peuvent gêner la prise en main.
- l'embase de l'aiguille comporte une chambre de visualisation du reflux sanguin ;
- l'embase de l'aiguille forme un logement tubulaire cylindrique situé dans l'axe de la portion rectiligne du tube d'aiguille et apte à être enfilé sur le tube protecteur pour coulisser sur le tube;
- l'extrémité arrière du tube protecteur est munie d'une pièce rigide arrière sur laquelle vient se clipper l'embase de l'aiguille dans ladite position arrière ;
- l'extrémité avant du tube protecteur est munie d'une pièce rigide avant qui définit un passage étroit pour ladite portion rectiligne du tube d'aiguille entre un logement ouvert vers l'arrière dans laquelle est reçue l'extrémité avant du tube protecteur et un logement ouvert vers l'avant ;
- l'embase d'aiguille, dans ladite position avant, est en appui sur ladite pièce rigide rapportée à l'extrémité avant du tube protecteur ;
- l'ensemble comprend une canule de longueur inférieure à la longueur de ladite portion rectiligne du tube d'aiguille et apte à glisser sur la portion rectiligne du tube d'aiguille, cette canule étant munie d'une embase apte à être fixée de façon détachable sur la pièce avant rapportée à l'extrémité avant du tube protecteur en sorte que l'extrémité biseautée du tube d'aiguille soit en saillie en avant de la canule lorsque l'embase d'aiguille est dans ladite position avant.

On décrira ci-après un exemple de réalisation d'un ensemble selon l'invention, en référence aux figures du dessin joint sur lequel :

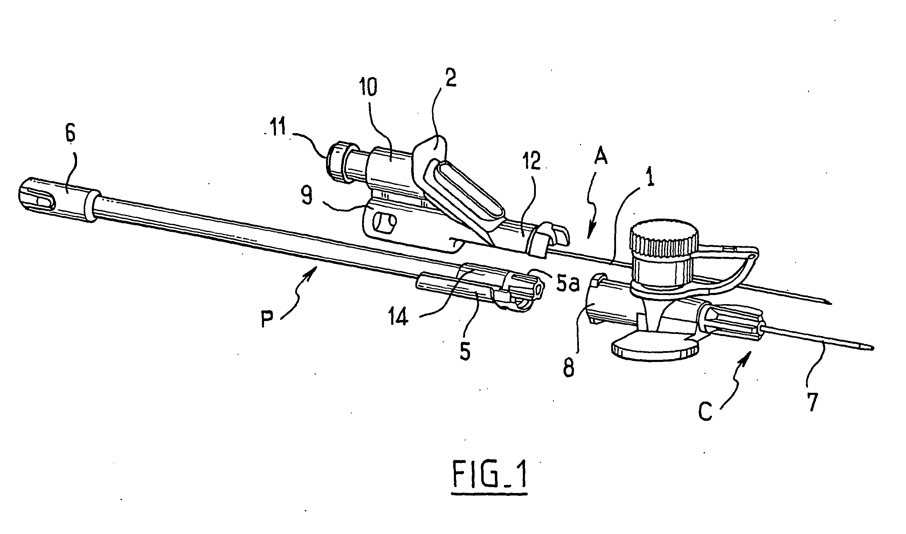

- la figure 1 est un schéma en perspective d'un ensemble dont on a représenté séparément l'aiguille, le protecteur d'aiguille muni de pièces accessoires et un cathéter court ;

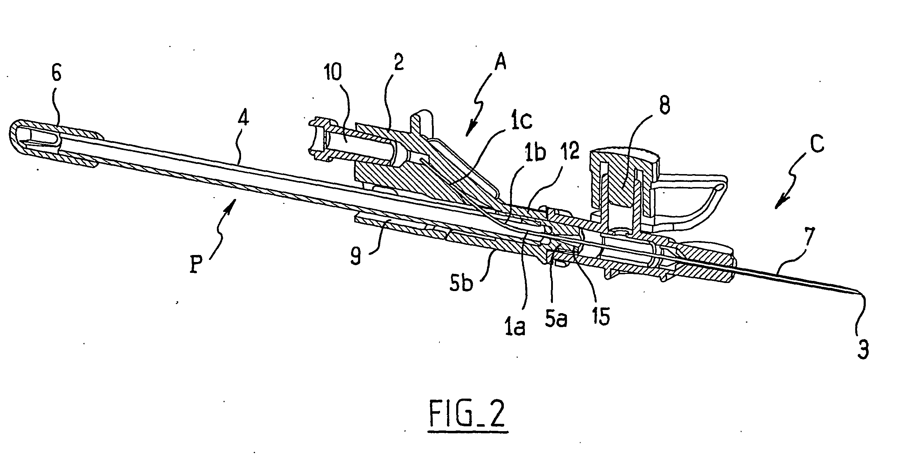

- la figure 2 est un schéma en coupe de l'ensemble de la figure 1 prêt à l'emploi ;
- la figure 3 est un schéma en coupe de l'ensemble au début de la ponction d'une veine ;
- la figure 4 est un schéma en coupe de l'ensemble au cours du retrait de l'aiguille ;
- la figure 5 est un schéma en coupe de l'ensemble après escamotage de l'aiguille ;
- la figure 6 est une vue en perspective agrandie d'un détail de la figure 5 ;
- la figure 7 est une vue en coupe du protecteur d'aiguille avec l'aiguille escamotée, et
- la figure 8 est une vue en perspective du protecteur d'aiguille contenant l'aiguille escamotée.

Sur les figures, on a représenté un ensemble qui comprend :
- une aiguille de ponction (A) constituée d'un tube d'aiguille (1) en acier inoxydable et d'une embase (2), le tube d'aiguille comprenant une portion de tube rectiligne (1a) terminée à son extrémité distale par un biseau (3) et dont l'extrémité opposée est reliée par une portion coudée (1b) à une autre portion du tube (1c) qui est oblique par rapport à la portion rectiligne et qui aboutit dans l'embase (2) de l'aiguille ;
- un tube protecteur (P) qui est un tube creux souple qui présente une fente longitudinale (4) et qui est muni à son extrémité avant d'une pièce rigide (5) et à son extrémité arrière d'une autre pièce rigide (6) ;
- un cathéter court (C) constitué d'une canule (7) qui présente une extrémité proximale munie d'une embase (8).

L'embase (2) de l'aiguille forme un logement tubulaire cylindrique (9) situé dans l'axe de la portion rectiligne (1a) du tube d'aiguille et apte à être enfilé sur le tube protecteur pour coulisser sur le tube. Elle forme également au dessus de ce logement une chambre (10) de visualisation du reflux sanguin qui communique avec la portion oblique (1c) du tube d'aiguille et qui est fermée par un bouchon air-vent (11). Elle forme également une avancée (12).

Le tube de l'aiguille est assemblé dans son embase par collage ou soudage ; il est cintré avant ou après cet assemblage.

La pièce arrière (6) est une bague qui ferme le tube protecteur. Elle servira de butée arrière pour fermer l'embase de l'aiguille et présente un ou deux tétons latéraux (16) destinés à coopérer avec une ou deux fenêtres (17) formées dans la paroi du logement cylindrique (9) de l'embase d'aiguille pour assurer le blocage de l'embase lorsque le biseau de l'aiguille est escamoté dans le tube protecteur.

La pièce avant est une pièce complexe destinée à coiffer l'extrémité avant du tube protecteur et qui forme :
- à l'avant, un nez (5a) destiné à être introduit dans l'embase (8) de la canule pour maintenir par coincement la canule sur cette pièce ;
- à l'arrière un logement cylindrique (14) apte à recevoir l'extrémité avant du tube protecteur en sorte que cette extrémité puisse être fixée adhésivement dans le logement ;
- sous le logement (14), un berceau (5b) apte à servir d'appui à l'embase de l'aiguille ;
- à l'intérieur de la pièce, un passage axial étroit (13) destiné à laisser passer la portion rectiligne (1a) de l'aiguille, ce passage débouchant à l'arrière dans le logement (14) et à l'avant dans une ouverture conique (15).

Le tube protecteur (P) est un tube en matière plastique qui présente une fente longitudinale sur toute la longueur du tube sauf à l'arrière sur quelques millimètres. La bague (6) est une bague rigide collée sur cette extrémité arrière.

On introduit le tube protecteur (P) dans le logement cylindrique (9) de l'embase de l'aiguille puis par dessus la portion coudée du tube d'aiguille : la fente du tube protecteur s'ouvre au passage de la portion coudée et se referme ensuite.

On enfile la pièce avant (5) sur la portion rectiligne du tube d'aiguille, en partant du biseau qui termine cette portion à l'avant, jusqu'à ce que le logement cylindrique (14) vienne coiffer l'extrémité avant fendu du tube protecteur et referme la fente. On assure éventuellement l'assemblage par collage.

On coince l'embase de la canule sur le nez de la la pièce avant (5).

L'embase de l'aiguille est mise en appui sur le berceau de la pièce (5).

L'ensemble est alors prêt à l'emploi.

Le tube protecteur peut être prédéformé pour faciliter la prise en main par l'utilisateur ou déformé élastiquement par l'utilisateur.

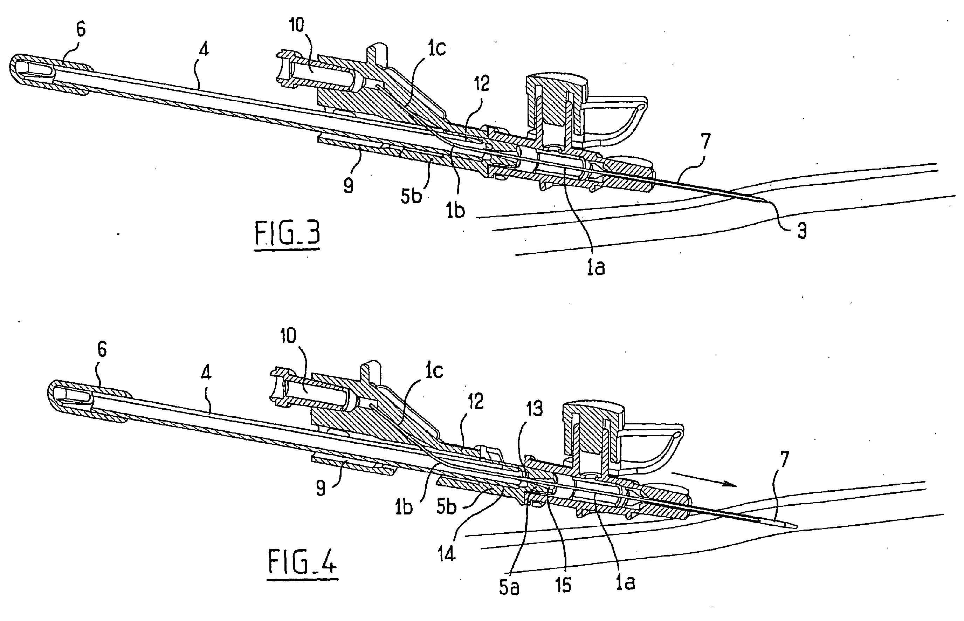

Ce dernier pique la veine du patient, attend le reflux sanguin lui confirmant le bon placement du biseau de l'aiguille dans la veine. Alors, il avance la canule en maintenant l'aiguille en place. Lorsque la canule avance, la pièce (5) et le tube protecteur avancent, mais l'embase de l'aiguille reste en place en arrière se séparant de l'embase de la canule (fig.4).

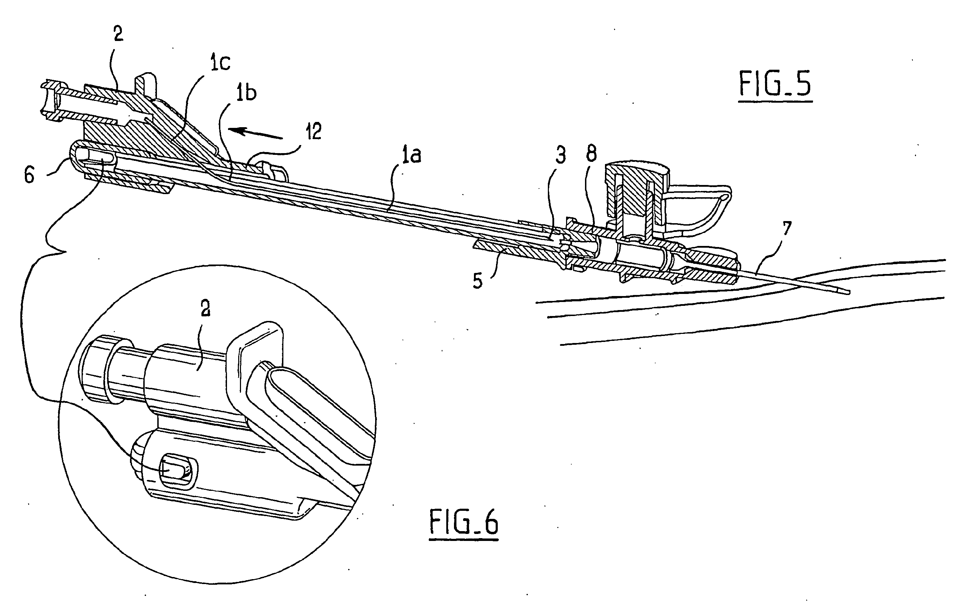

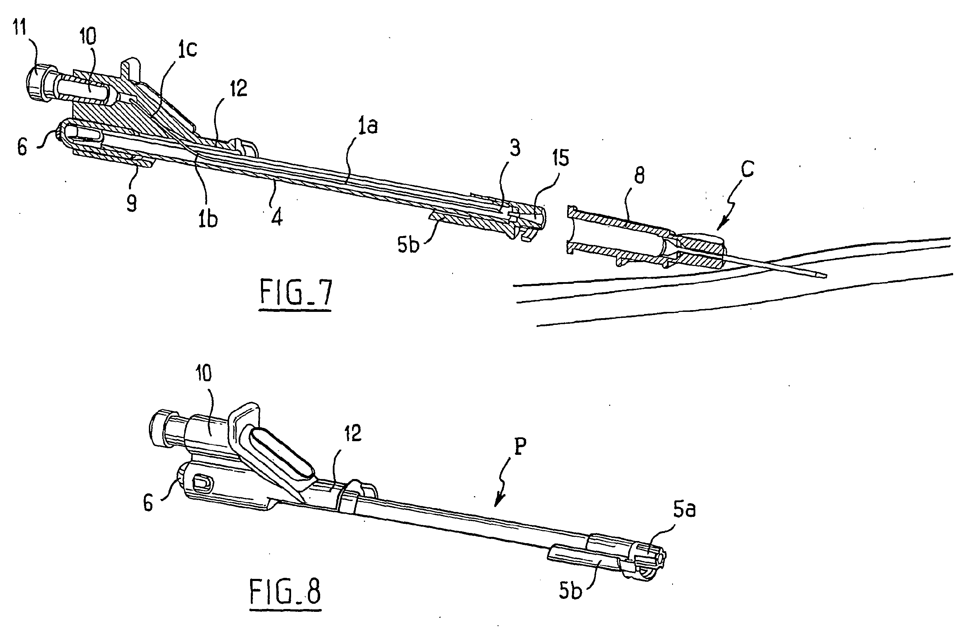

Lorsque la canule est avancée complètement, l'utilisateur bloque alors manuellement l'embase de la canule, tire sur l'embase de l'aiguille pour la faire coulisser sur le tube protecteur jusqu'à arriver en butée sur la bague arrière (6) du tube protecteur (fig. 5), avec clippage de l'embase d'aiguille sur la butée arrière, et tire un peu plus fort alors pour déconnecter la canule (fig.7).

Lorsque l'aiguille est tirée en arrière, la portion d'aiguille coudée recule dans la fente du tube protecteur en l'ouvrant au fur et à mesure. L'élasticité du tube amène la fente à se refermer sur elle-même après le passage du coude. L'aiguille, lors de ce retrait redresse le tube protecteur s'il a été déformé dans une phase précédente.

Lorsque l'embase de l'aiguille arrive en butée sur la bague du tube protecteur, le biseau du tube d'aiguille s'est dégagé de son logement dans la pièce avant (5) de 1 ou de plusieurs dixièmes de millimètres. Il est alors très difficile de le réengager dedans car le diamètre du passage interne (13) de la pièce est ajusté (très peu de jeu), un cône inverse à l'entrée de la pièce gêne la réintroduction du tube d'aiguille. Le tube protecteur, qui est seul lié à la pièce avant et qui guide le tube est déformable et rend difficile le centrage du tube dans la pièce avant surtout en le tenant par l'embase d'aiguille.

Enfin, il est possible d'avoir une certaine élasticité en traction sur le tube protecteur, lors de la traction pour déconnecter la coiffe de la canule. Il peut s'allonger un peu. Lors du relâchement et avec la coiffe déconnectée le biseau revient un peu vers la coiffe et est dévié par le cône interne de celle-ci.

Enfin, le clippage entre la bague et l'embase d'aiguille lorsque l'embase est en butée arrière empêche toute solidarisation ultérieure et donc tout mouvement avant dans le sens de la réintroduction (fig.6)

Au final, on a une aiguille protégée par le tube fendu (pas de contact direct possible avec le tube d'aiguille (sang, ...)) et bloquée dans celui-ci entre la bague et la coiffe. La coiffe rigide, est un piège pour le biseau et empêche tout risque de piqûre.
L'invention n'est pas limitée à la réalisation qui a été décrite.

## Revendications

1. Ensemble comprenant une aiguille de ponction (A) constituée d'un tube d'aiguille (1) dont une extrémité (3) est biseautée et d'une embase (2) solidaire de l'autre extrémité du tube d'aiguille, et un tube protecteur (P) pour cette aiguille, ce tube protecteur présentant une fente longitudinale (4) qui permet de faire coulisser une partie de l'aiguille dans le tube protecteur en actionnant une partie de l'aiguille extérieure au tube, **caractérisé en ce que** le tube d'aiguille (1) comporte une portion de tube rectiligne (1a) dont une extrémité (3) est biseautée et dont l'autre extrémité se raccorde par une portion coudée (1b) à une portion du tube oblique (1c) qui aboutit dans l'embase d'aiguille (2), et **en ce que** le tube protecteur est apte à loger intégralement ladite portion rectiligne (1a) du tube d'aiguille, ladite embase (2) de l'aiguille étant intégralement située à l'extérieur du tube protecteur et montée à coulisse sur le tube protecteur, entre une position avant où ladite portion rectiligne (1a) est en saillie en avant du tube protecteur et une position arrière où ladite portion rectiligne (1a) est contenue dans le tube protecteur, ladite fente longitudinale (4) du tube protecteur s'étendant jusqu'à l'extrémité avant du tube protecteur et étant à ouverture élastique de façon à s'ouvrir pour permettre le passage de la portion coudée (1b) du tube d'aiguille au travers de la fente et à se refermer après ce passage.

2. Ensemble selon la revendication 1 dont le tube protecteur est déformable pour ne pas gêner la prise en main de l'ensemble lors de la ponction et il est muni à l'avant d'une pièce rapportée rigide pour son raccordement à l'embase du cathéter.

3. Ensemble selon la revendication 2 dont ladite pièce rigide avant (5) définit un passage axial étroit (13) pour ladite portion rectiligne du tube d'aiguille entre un logement (14) ouvert vers l'arrière et qui reçoit l'extrémité avant du tube protecteur et un logement (15) ouvert vers l'avant.

4. Ensemble selon l'une des revendications 2 et 3 dans lequel l'extrémité arrière du tube protecteur est munie d'une pièce rigide arrière (6) sur laquelle vient se clipper l'embase (2) de l'aiguille dans ladite position arrière.

5. Ensemble selon l'une des revendications 2 à 4 dans lequel l'embase d'aiguille (2) est en appui sur ladite pièce rigide avant dans ladite position avant.

6. ensemble selon l'une des revendications 1 à 5 et dont ladite embase d'aiguille comporte une chambre (10) de visualisation du reflux sanguin.

7. Ensemble selon l'une des revendications 1 à 6 et dont l'embase (2) de l'aiguille forme un logement tubulaire cylindrique (9) situé dans l'axe de la portion rectiligne (1a) du tube d'aiguille et apte à être enfilé sur le tube protecteur pour coulisser sur le tube.

8. Ensemble selon l'une des revendications 2 à 7 et qui comprend une canule (7) de longueur inférieure à la longueur de ladite portion rectiligne (1a) du tube d'aiguille et apte à glisser sur cette portion rectiligne du tube d'aiguille, cette canule étant munie d'une embase (8) apte à être fixée de façon détachable sur ladite pièce avant (5) en sorte que l'extrémité biseautée (3) du tube d'aiguille soit en saillie en avant de la canule lorsque l'embase d'aiguille est dans ladite position avant.

## Claims

1. An assembly comprising an aspiration needle (A) consisting of a needle tube (1), one end of which is bevelled, and a base (2) integral with the other end of the needle tube, and a protective tube (P) for this needle, this protective tube having a longitudinal slot (4) which allows a portion of the needle to slide in the protective tube by actuating a portion of the needle external to the tube, **characterized in that** the needle tube (1) includes a rectilinear tube portion (la), one end (3) of which is bevelled and the other end of which connects through a bent portion (1b) to a portion of the oblique tube (1c) which ends up in the needle base (2), and **in that** the protective tube is able to entirely accommodate said rectilinear portion (1a) of the needle tube, said base (2) of the needle being entirely located outside the protective tube and slidably mounted on the protective tube between a front position where said rectilinear portion (1a) protrudes in front of the protective tube and a rear position where said rectilinear portion (1a) is contained in the protective tube, said longitudinal slot (4) of the protective tube extending right up to the front end of the protective tube and having an elastic opening so as to open in order to allow the bent portion (1b) of the needle tube to pass through the slot and to close after this passing.

2. The assembly according to claim 1, the protective tube of which is deformable so as not to interfere with the handling of the assembly during aspiration and it is provided at the front with a rigid insert for connecting it to the base of the catheter.

3. The assembly according to claim 2, said front rigid insert (5) of which defines a narrow axial passage for said rectilinear portion of the needle tube between a housing (14) open towards the rear and which receives the front end of the protective tube and a housing (15) open towards the front.

4. The assembly according to any of claims 2 and 3, wherein the rear end of the protective tube is provided with a rear rigid insert (6) onto which the base (2) of the needle in said rear position will be fastened by a clip.

5. The assembly according to any of claims 2 to 4, wherein the needle base (2) is supported on said front rigid insert in said front position.

6. The assembly according to any of claims 1 to 5 and said needle base of which includes a chamber (10) for viewing backflow of blood.

7. The assembly according to any of claims 1 to 6 and the base (2) of the needle of which forms a cylindrical tubular housing (9) located in the axis of the rectilinear portion (1a) of the needle tube and capable of being slipped onto the protective tube in order to slide on the tube.

8. The assembly according to any of claims 2 to 7 and which comprises a cannula (7) with a length less than the length of said rectilinear portion (1a) of the needle tube and capable of sliding on this rectilinear portion of the needle tube, this cannula being provided with a base (8) capable of being removably attached on said front part (5) so that the bevelled end (3) of the needle tube protrudes forwards from the cannula when the needle base is in said front position.

## Patentansprüche

1. Anordnung, folgendes umfassend: eine Punktionsnadel (A), die aus einer Nadelröhre (1), deren eines Ende (3) abgeschrägt ist, und einem Aufnahmeteil (2), das fest mit dem anderen Ende der Nadelröhre verbunden ist, gebildet wird, und eine Schutzröhre (P) für diese Nadel, wobei diese Schutzröhre einen Längsschlitz (4) hat, der es gestattet, einen Teil der Nadel in der Schutzröhre zu verschieben, indem ein außerhalb der Röhre liegender Teil der Nadel betätigt wird, **dadurch gekennzeichnet, dass** die Nadelröhre (1) einen geradlinigen Röhrenabschnitt (1a) umfasst, dessen eines Ende (3) abgeschrägt ist und dessen anderes Ende über einen gekrümmten Abschnitt (1 b) mit einem schrägen Röhrenabschnitt (1c) verbunden ist, der in das Aufnahmeteil der Nadel (2) mündet, und dadurch, dass die Schutzröhre dafür geeignet ist, den geradlinigen Abschnitt (1a) der Nadelröhre vollständig aufzunehmen, wobei das Aufnahmeteil (2) der Nadel sich vollständig außerhalb der Schutzröhre befindet und gleitend verschiebbar auf der Schutzröhre angebracht ist, und dies zwischen einer vorderen Position, in der der geradlinige Abschnitt (1a) vorne aus der Schutzröhre hervorsteht, und einer hinteren Position, in der der geradlinige Abschnitt (1a) in der Schutzröhre enthalten ist, wobei der Längsschlitz (4) der Schutzröhre sich bis zum vorderen Ende der Schutzröhre erstreckt und eine elastische Öffnung hat, die so eingerichtet ist, dass er sich öffnet, um den Durchgang des gekrümmten Abschnitts (1 b) der Nadelröhre entlang des Schlitzes zu gestatten und dass er sich nach diesem Durchgang wieder verschließt.

2. Anordnung nach Anspruch 1, deren Schutzröhre verformbar ist, um das Greifen der Anordnung bei der Punktion nicht zu behindern, wobei sie vorne mit einem steifen aufgesetzten Teil ausgestattet ist, das ihrem Anschluss an ein Aufnahmeteil des Katheters dient.

3. Anordnung nach Anspruch 2, deren vorderes steifes Teil (5) zwischen einer Aufnahme (14), die nach hinten offen ist und die das vordere Ende der Schutzröhre aufnimmt, und einer Aufnahme (15), die nach vorne offen ist, einen engen axialen Durchgang (13) für den geradlinigen Abschnitt der Nadelröhre definiert.

4. Anordnung nach einem der Ansprüche 2 und 3, bei der das hintere Ende der Schutzröhre mit einem hinteren steifen Teil (6) ausgestattet ist, auf dem das Aufnahmeteil (2) der Nadel in der hinteren Position festgeklippt wird.

5. Anordnung nach einem der Ansprüche 2 bis 4, bei der das Nadelaufnahmeteil (2) in der vorderen Position auf dem vorderen steifen Teil zur Abstützung kommt.

6. Anordnung nach einem der Ansprüche 1 bis 5, bei der die Nadelaufnahme eine Kammer (10) zur Anzeige des Blutrückflusses umfasst.

7. Anordnung nach einem der Ansprüche 1 bis 6, bei der das Aufnahmeteil (2) der Nadel eine zylindrische röhrenförmige Aufnahme (9) bildet, die sich in der Achse des geradlinigen Abschnitts (1a) der Nadelröhre befindet und die dafür geeignet ist, auf die Schutzröhre geschoben zu werden, um auf der Röhre zu gleiten.

8. Anordnung nach einem der Ansprüche 2 bis 7, die eine Kanüle (7) umfasst mit einer Länge, die kleiner ist als die Länge des geradlinigen Abschnitts (1a) der Nadelröhre und die dafür geeignet ist, auf diesem geradlinigen Abschnitt der Nadelröhre zu gleiten, wobei diese Kanüle mit einem Aufnahmeteil (8) ausgestattet ist, das dafür geeignet ist, abnehmbar auf dem vorderen Teil (5) befestigt zu werden, und dies derart, dass das abgeschrägte Ende (3) der Nadelröhre vorne aus der Kanüle hervorsteht, wenn das Nadelaufnahmeteil in der vorderen Position ist.
